# EUROPEAN PATENT SPECIFICATION

(11) **EP 3 527 150 B1**
(45) Date of publication and mention of the grant of the patent: **01.04.2020**
(21) Application number: 18156965.8
(22) Date of filing: 15.02.2018
(51) Int. Cl.: A61B 17/29

(54) **MEDICAL INSTRUMENT**
MEDIZINISCHES INSTRUMENT
INSTRUMENT MÉDICAL

(43) Date of publication of application: 21.08.2019
(73) Proprietor: University of Dundee, Dundee DD1 4HN (GB)
(72) Inventor: Coleman, Stuart, Dundee, DD1 4HN (GB); Martin, James Duncan S., Dundee, DD1 4HN (GB); Ross, Pete, Dundee, DD1 4HN (GB)
(74) Representative: Hofmeister, Frank

(56) References cited:
- EP-A1- 3 106 097
- DE-U1- 9 300 161
- FR-A1- 2 681 775
- US-A- 2 028 635
- US-A- 5 827 323

## Description

The invention relates to a medical instrument having a shank, a tool which is arranged on the distal end of the shank and consists of two pivotable jaw parts, wherein the two pivotable jaw parts can additionally be angled in relation to a longitudinal axis of the shank, and having a handle which is arranged on the proximal end of the shank, wherein two separate drives are arranged on the handle, wherein a first drive for pivoting a first jaw part relative to the second jaw part is designed as a grip part, which is mounted pivotable on the handle and is operatively connected to the first jaw part of the tool via of a pull/push rod, which is mounted in an axially displaceable manner in the shank, and, that the second drive for angling the two jaw parts in relation to the longitudinal axis of the shank .

Generic medical instruments are used, for example, in surgery in the ENT field, in particular in paranasal sinus surgery. In the case of said surgery, the surgeons frequently use several pairs of forceps which are similar to one another and only differ from one another in the respective access angle.

A generic medical instrument is disclosed for example, in DE 10 2011 008 013 A1. In the case of said disclosed medical instrument, the two jaw parts are pivotable independently from one another and relative to one another and can additionally be angled in relation to the longitudinal axis of the instrument. Certainly, said configuration has definitely proved its worth in practice, it is preferable, however, in the case of individual surgery when the pivotability of the jaw parts includes further angular ranges. Patent US 5,827,323 discloses another similar example of a known instrument with two pivotable jaw parts.

Proceeding from this, the problem addressed by the invention is to create a medical instrument of the aforementioned type which while having a simple design is versatilely useable.

According to the invention the solution to this problem is characterized in that the pivoting of the two jaw parts relative to one another and the angling of the jaw parts in relation to the longitudinal axis of the shank are effected about the same pivot axis, wherein the pivot axis is arranged eccentrically with respect to the longitudinal axis of the shank.

As a result of displacing the pivot axis, which is common to all the movements of the jaw parts, out of the longitudinal axis of the shank toward an edge region of the shank, it is possible to widen the pivot angle of the two jaw parts to greater than 90° when angling in relation to the longitudinal axis of the instrument. Said measure provides the surgeon with a larger scope of application for an instrument designed in such a manner without having to change to another instrument during surgery. Additionally, offsetting the pivot axis allows for a stronger instrument compared to one with a central pivot. Furthermore, it is proposed by the invention that a rod which is arranged in the shank is fixedly connected to the handle on its proximal end and provides a fixed pivot about which a second jaw part may pivot on its distal end.

According to a practical embodiment of the invention, it is proposed that the distal end of the shank, designed in a bifurcated manner, consists of two webs which are arranged parallel to one another and at a distance from one another, wherein the pivot axis is realized as an axle pin which is mounted in both webs. It is precisely the open configuration of the distal end of the shank in which the pivot axis is mounted eccentrically that makes the large pivot angle possible when angling the jaw parts of the tool in relation to the longitudinal axis of the instrument.

In order to be able to adjust the pivotable jaw parts of the tool individually and at a large pivot angle, it is proposed by the invention that the jaw parts are coupled in each case via coupling levers with the associated pull/push rod respectively rod. The coupling levers, which are interposed between the pivotable jaw parts and the associated rods, enlarge the freedom of movement of the jaw parts compared to a direct bearing arrangement of the jaw parts on the respective rods.

For designing the pull/push rod which serves for the drive of the pivotable first jaw part, it is proposed with the invention that the pull/push rod and the rod are arranged parallel side by side in the shaft and that both rods arranged parallel side by side together comprise a circular cross section. Said realizations of the two pull/push rods enable a space-saving design which also allows the use in very narrow shank diameters.

Each, pull/push rod and rod, is advantageously designed in a semi-circular manner in cross section in order to provide both rods with the same stability.

It is proposed according to the invention with a practical embodiment for designing the pull/push rod and the rod that the rod which is fixedly connected to the handle is mounted in a recess respectively flat area of the pull/push rod which is coupled with the adjustable grip part. As a result of said configuration, the pull/push rod provided with the recess forms a guiding bearing arrangement for the other rod.

According to a preferred embodiment of the invention, it is proposed that the drive for angling the two jaw parts in relation to the longitudinal axis of the shank is operatively connected to both jaw parts of the tool via the shank tube which is mounted in an axially displaceable manner relative to the handle.

In addition, it is proposed by the invention that the shank tube is operatively connected to the adjusting wheel , which in turn is coupled to the handle via a control journal. The elongated hole in the pull/push rod which is coupled with the pivotable grip part enables the independent actuation of the pull/push rod relative to the other rod.

Finally, it is proposed by the invention that, for converting the rotational movement of the adjusting wheel into the axial movement of the shank tube, the control journal, which is connected fixedly to the handle, is mounted in a guiding manner in a helical guide track, wherein the helical guide track is formed in a sleeve which is securable in the adjusting wheel.

Further features and advantages of the invention emerge with reference to the associated drawings, in which an exemplary embodiment of a medical instrument according to the invention is illustrated only by way of an example without restricting the invention to said exemplary embodiment. In the drawings:
- Fig. 1: shows a schematic side view of a medical instrument according to the invention;
- Fig. 2: shows a part exploded drawing of the medical instrument according to Fig. 1, but without the handle on the proximal side;
- Fig. 3: shows a perspective view of the detail III according to Fig.2 in the state put together, but without the shank tube;
- Fig. 4: shows an exploded drawing of the detail IV according to Fig.3;
- Fig. 5: shows a top view of the pull/push rods according to Fig. 3 in the disassembled state;
- Fig. 6: shows a side view of the pull/push rods according to Fig. 5;
- Fig. 7: shows sections along the lines VII-VII according to Fig. 5 and
- Fig. 8: shows a longitudinal section through the detail VIII according to Fig. 2.

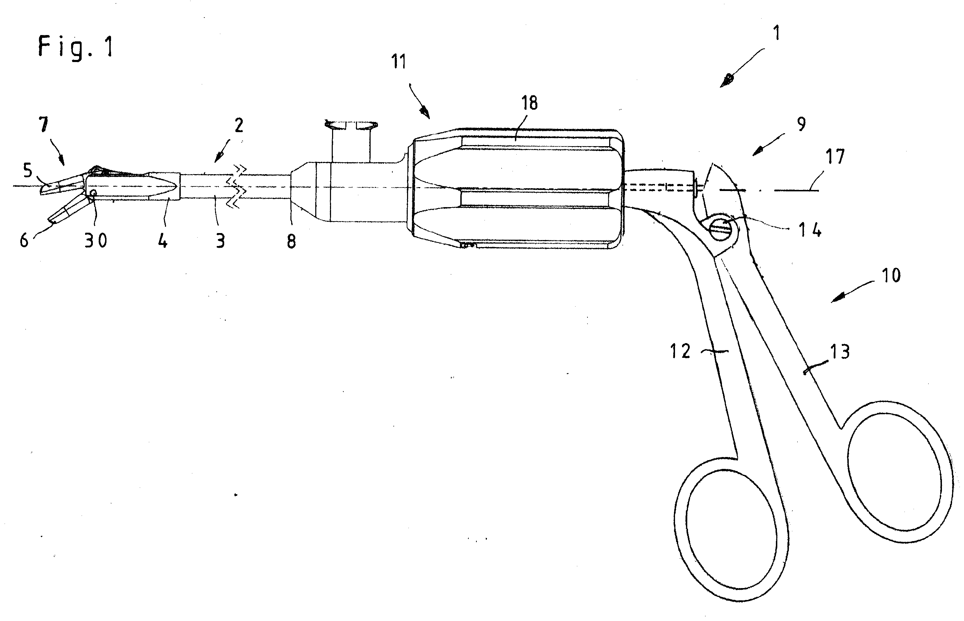

Figure Fig. 1 shows a medical instrument 1 which is designed as a pair of forceps, as used, for example in surgery in the ENT field, in particular in paranasal sinus surgery.

The medical instrument 1 illustrated essentially consists of a shank 2 with a hollow shank tube 3, at the distal end 4 of said shank 2 a tool 7 consisting of two pivotable jaw parts 5 and 6 is arranged and at the proximal end 8 of said shank 2 a handle 9 is arranged. The pivotable jaw parts 5 and 6 of the tool 7 are actuatable via separate drives 10 and 11 which are arranged on the handle 9. Drive 10 will pivot only jaw 6 while drive 11 will pivot both jaw parts 5 and 6.

In the embodiment of the medical instrument 1 shown in Fig. 1, the handle 9 comprises two grip parts 12 and 13, wherein the grip part 12 is formed integrally and rigidly with the handle 9 and the other grip part 13 being mounted on the handle 9 so as to be pivotable about a pivot axis 14 in relation to the rigid grip part 12.

The pivotable grip part 13 forms the drive 10 for the jaw part 6 of the tool 7. For this purpose, the pivotable grip part 13 and the jaw part 6 are operatively connected together via a pull/push rod 15 in such a manner that by pivoting of the grip part 13, the jaw part 6 is transferred from a closed position into an open position via the pull/push rod 15 which is mounted in an axially-displaceable manner in the hollow shank tube 3 of the shank 2.

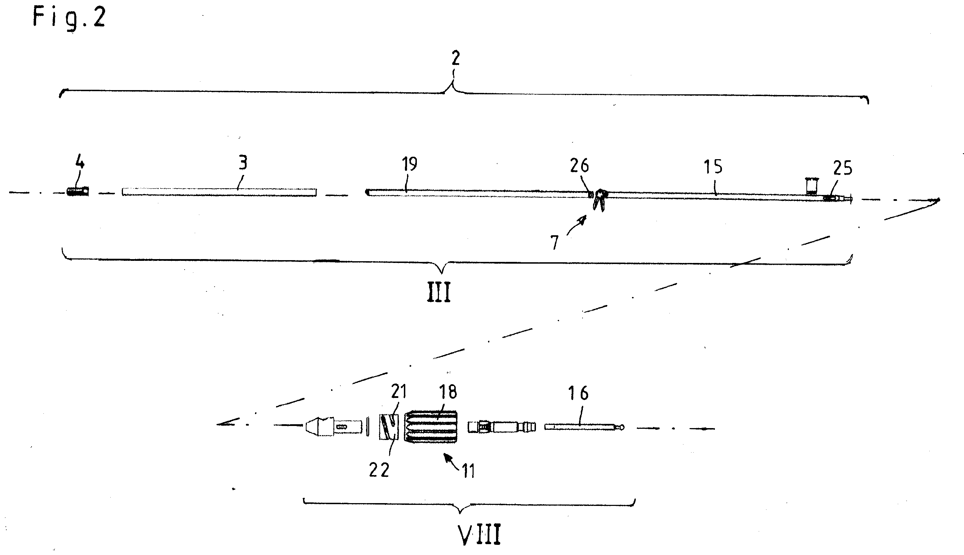

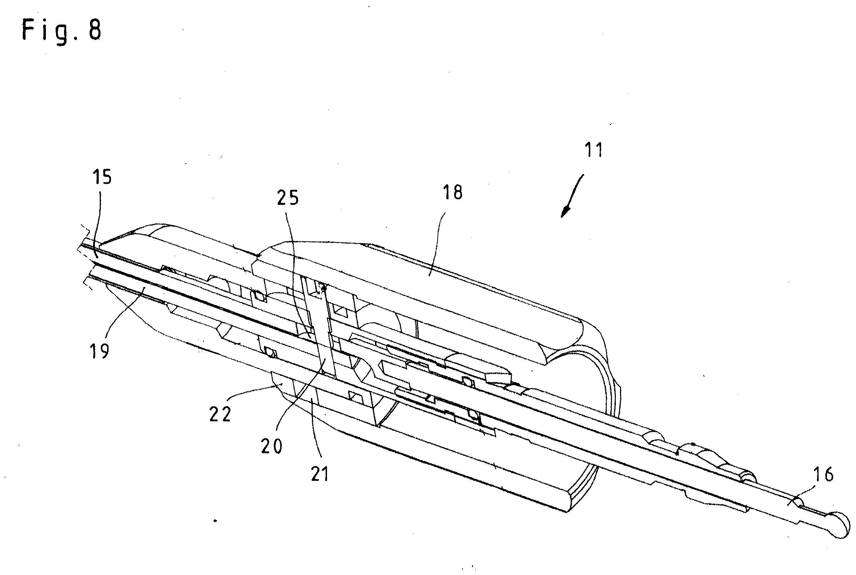

As can be seen from Fig. 2 and Fig. 8, the pivotable grip part 13 is coupled via a coupling rod 16, which is mounted in the handle 9, in a play-free manner with the pull/push rod 15 which is mounted in the hollow shank tube 3 of the shank 2.

The drive 11 allows the pivoting of both jaw parts 5 and 6 simultaneously. This means that the displacement of grip part 13 is dependent only on the degree of openness of the jaw parts 5 and 6, i.e. the angle between pivotable jaw parts 5 and 6. This is advantageous compared to two independently controllable jaw parts, as operation is more intuitive as the user can determine approximately how far open the jaw parts 5 and 6 are based on the position of their hand on grip part 13, and is ergonomically superior as the total motion of grip part 13 is more limited, so that the users hand may remain in a comfortable position on the handle 9. Also, this control configuration allows for the rotation of both jaw parts 5 and 6 while their position relative to each other remains near-constant, which may be useful while manipulating objects with the tool 7, this would be difficult to achieve with independently controlled jaw parts.

Pivoting of both jaw parts 5 and 6 is achieved by applying drive 11 to an adjusting wheel 18 which is rotatable about the longitudinal axis 17 of the shank 2. In order to actuate the jaw parts 5 and 6, the adjusting wheel 18 is operatively connected to the jaw parts 5 and 6 via the shank tube 3 which is mounted in an axially displaceable manner relative to the handle 9. A rod 19 is operatively fixedly connected to the handle 9 respectively to the rigid grip part 12, and the push/pull rod 15 is operatively connected to the pivotable grip part 13. Therefore, push/pull rod 15 and rod 19 provide a fixed base about which both articulatable jaw parts 5 and 6 pivot when the shank tube 3 is axially displaced.

A control journal 20 is operatively fixedly connected to the handle 9 and engages in a helical guide track 21 that is arranged in the adjusting wheel 18. The adjusting wheel 18 is axially fixed to the shank tube 3. This allows the rotational movement of the adjusting wheel 18 about the longitudinal axis 17 to be converted into the axial displacement of both the adjusting wheel and the shank tube 3, which in turn causes jaw parts 5 and 6 to pivot. The jaw parts 5 and 6 will pivot in the same direction and by the same angle so that their degree of openness will not change when drive 11 is applied. In the embodiment illustrated, the helical guide track 21 is arranged in a sleeve 22 which is fixed in the adjusting wheel 18.

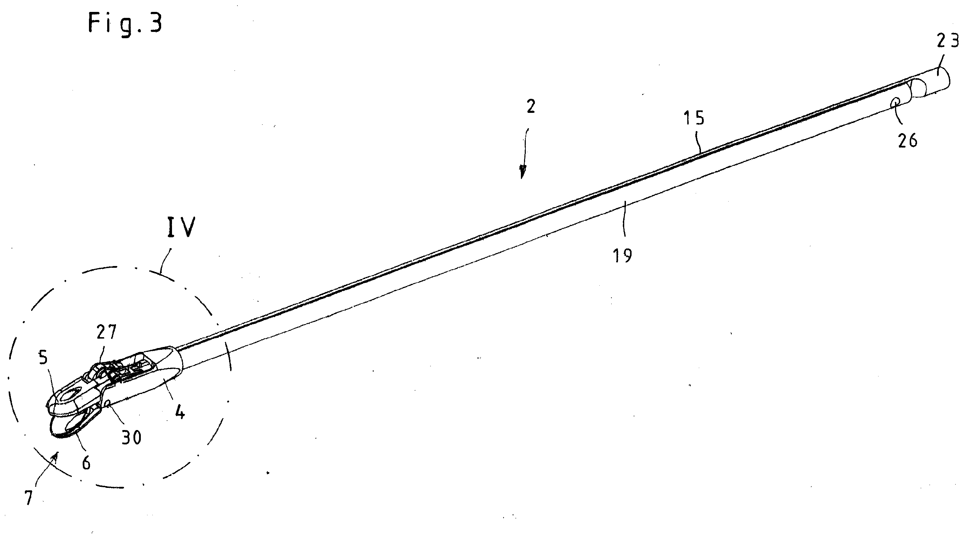

As can be seen from Fig. 3 and Figs. 5 to 7, in the case of the illustrated embodiment the pull/push rod 15, which is operatively connected to the pivotable grip part 13, comprises a portion 23 with a circular cross section on the proximal end for the connection to the coupling rod 16. From the portion 23 distally, the pull/push rod 15 comprises a flat area 24, in which the pull/push rod 15 has a semi-circular cross section.

The rod 19, which is coupled to the handle 9 via control journal 20, is designed in a semi-circular manner in cross section over its entire length.

With the medical instrument 1 in the mounted state, the rod 19 which is fixedly coupled with the handle 9 is mounted in the flat area 24 of the pull/push rod 15 which is coupled with the pivotable grip part 13, such that the pull/push rod 15 and fixed rod 19, which are arranged parallel side by side, together have a circular cross section, as can be seen from Fig. 3. Said embodiments of the two rods 15 and 19 enable a space-saving compact design which also allows the use in very narrow shank diameters.

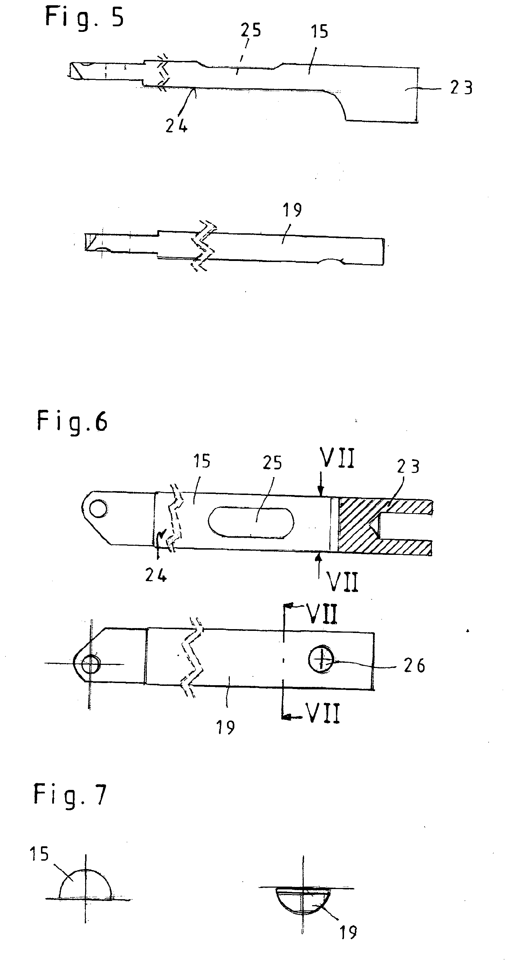

As can be seen additionally from Figs. 5, 6 and 8, an elongated hole 25 is formed in the pull/push rod 15 for the passage and axial movement of the control journal 20 which is mounted in a bore 26 in rod 19 so that it fixedly connects rod 19 to the handle 9. The axial displacement of the pull/push rod 15, which is independent of the pull/push rod 19, is made possible only as a result of said elongated hole 25.

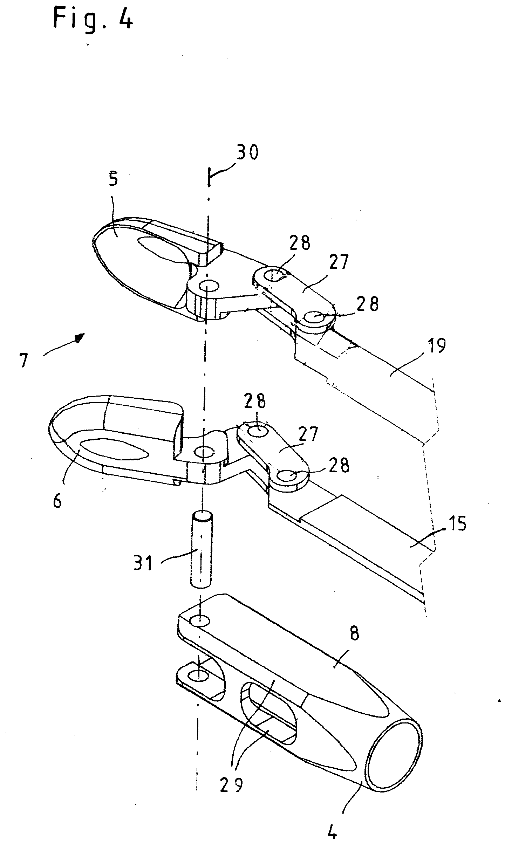

At the distal end, the pull/push rod 15 and rod 19 are connected in each case to the respective jaw parts 6 and 5 via a coupling lever 27, wherein each coupling lever 27 being pivotably mounted in each case, both on the associated pull/push rod 15 or rod 19 and on the associated jaw part 6 or 5, via a bolt 28, as can be seen from Figs. 3 and 4.

In addition to the pivoting of the jaw parts 5 and 6 relative to one another, the jaw parts 5 and 6 of the tool 7 are additionally designed so they can be angled in relation to the longitudinal axis 17 of the shank 2 via drive 11.

As can be seen additionally from Figs. 3 and 4, the distal end 4 of the shank 2 is realized in a bifurcated manner and consists of two webs 29 which are arranged parallel to one another and at a distance from one another. The coupling levers 27, which connect the pull/push rods 15 and 19 to the jaw parts 5 and 6, are mounted between the webs 29 of the distal end 4 of the shank 2.

The pivoting of the jaw part 5 relative to jaw part 6 and the angling of the jaw parts 5 and 6 in relation to the longitudinal axis 17 of the shank 2 are effected about one single pivot axis 30, said pivot axis 30, which is common to both movements of the jaw parts 5 and 6, being arranged eccentrically with respect to the longitudinal axis 17 of the shank 2.

The pivot axis 30 itself is realized, in the case of the illustrated embodiment, as an axle pin 31 which is mounted in both webs 29 of the distal end 4 of the shank 2 and in both jaw parts 5 and 6.

As a result of displacing the pivot axis 30, which is common to all the movements of the jaw parts 5 and 6, out of the longitudinal axis 16 of the shank 2 toward an eccentric edge region of the shank 2, it is possible to widen the pivot angle of the two jaw parts 5 and 6 to more than 90° when angling in relation to the longitudinal axis 16 of the shank 2. Said measure provides the surgeon with a larger scope of application for a medical instrument 1 that is designed in such a manner without having to change to a different instrument during the surgery. Additionally, this can allow for a larger lateral distance between the pivot axis 30 and the linkage to the push/pull rod 15 and rod 19, which can reduce forces in the linkage and allow for a stronger or smaller instrument design.

The bifurcated construction of the distal end 4 of the shank 2 with the two webs 28 which are arranged parallel to one another and at a distance from one another enables the large pivot angle when angling the jaw parts 5 and 6 of the tool 7 in relation to the longitudinal axis 16 of the shank 2, precisely as a result of the open configuration of the distal end 4 of the shank 2 in which the pivot axis 30 is eccentrically mounted. Simultaneously a compact design of the medical instrument 1 is achieved.

A medical instrument 1 which is designed as described beforehand is characterized in that it is versatilely useable with a simple design and provides the surgeon with a large scope of application with only one instrument. The scope of the medical instrument 1 as an invention, is defined by the claims appended hereafter.

### List of reference numbers

- 1: Medical instrument
- 2: Shank
- 3: Shank tube
- 4: Distal end (shank)
- 5: Pivotable jaw part
- 6: Pivotable jaw part
- 7: Tool
- 8: Proximal end (shank)
- 9: Handle
- 10: Drive
- 11: Drive
- 12: Grip part (rigid)
- 13: Grip part (pivotable)
- 14: Pivot axis
- 15: Pull/push rod
- 16: Coupling rod
- 17: Longitudinal axis
- 18: Adjusting wheel
- 19: rod
- 20: Control journal
- 21: Guide track
- 22: Sleeve
- 23: Portion
- 24: Flat area
- 25: Elongated hole
- 26: Bore
- 27: Coupling lever
- 28: Bolt
- 29: Web
- 30: Pivot axis
- 31: Axle pin

## Claims

1. Medical instrument having a shank (2), a tool (7) which is arranged on the distal end (4) of the shank (2) and consists of two pivotable jaw parts (5, 6), wherein the pivotable jaw parts (5, 6) can additionally be angled in relation to a longitudinal axis (17) of the shank (2), and having a handle (9) which is arranged on the proximal end (8) of the shank (2), wherein two separate drives (10, 11) are arranged on the handle (9), wherein a first drive (10) for pivoting a first jaw part (6) relative to the second jaw part (5) is designed as a grip part (13) which is mounted pivotable on the handle (9) and is operatively connected to the first pivotable jaw part (6) of the tool (7) via a first pull/push rod (15) which is mounted in an axially displaceable manner in the shank (2), wherein the pivoting of the two jaw parts (5 and 6) relative to one another and the angling of the jaw parts (5 and 6) in relation to the longitudinal axis (17) of the shank (2) are effected about the same pivot axis (30), wherein the pivot axis (30) is arranged eccentrically with respect to the longitudinal axis (17) of the shank (2)
**characterized in that**
the second drive (11) for angling the two jaw parts (5, 6) in relation to the longitudinal axis (17) of the shank (2) is operatively connected to the second jaw part (5) of the tool (7) via a second rod (19) which is mounted in an axially displaceable manner in the shank (2), and wherein,
the rod (19) which is arranged in the shank (2) is fixedly connected to the handle (9) on its proximal end and provides a fixed pivot about which second jaw part (5) may pivot on its distal end and **in that** the pull/push rod (15) and rod (19) are arranged parallel side by side in a hollow shank tube (3) of the shank (2), and **in that** the two rods (15 and 19) arranged parallel side by side together comprise a circular cross section.

2. Medical instrument according to Claim 1, **characterized in that** the distal end (4) of the shank (2), designed in a bifurcated manner, consists of two webs (29) which are arranged parallel to one another and at a distance from one another, wherein the pivot axis (30) is realized as an axle pin (31) which is mounted in both webs (29).

3. Medical instrument according to Claim 1, **characterized in that** the pivotable jaw parts (5 and 6) are coupled in each case via coupling levers (27) with the respectively associated pull/push rod (15) and rod (19).

4. Medical instrument according to Claim 1, **characterized in that** each pull/push rod (15) and rod (19), is designed in a semi-circular manner in cross section.

5. Medical instrument according to Claim 1, **characterized in that** the rod (19) which is fixedly connected to the handle (2) is mounted in a flat area (24) of the pull/push rod (15) which is coupled with the pivotable grip part (13).

6. Medical instrument according to one of Claims 1 to 5, **characterized in that** the drive (11) for angling the two jaw parts (5, 6) in relation to the longitudinal axis (17) of the shank (2) is designed as an adjusting wheel (18) which is rotatable about the longitudinal axis (17) of the shank (2).

7. Medical instrument according to one of Claims 1 to 6, **characterized in that** the drive (11) for angling the two jaw parts (5, 6) in relation to the longitudinal axis (17) of the shank (2) is operatively connected to both jaw parts (5, 6) of the tool (7) via the shank tube (3) which is mounted in an axially displaceable manner relative to the handle (9).

8. Medical instrument according to Claim 7, **characterized in that** the shank tube (3) is operatively connected to the adjusting wheel (18), which in turn is coupled to the handle (9) via a control journal (20).

9. Medical instrument according to Claim 8, **characterized in that** for converting the rotational movement of the adjusting wheel (18) into the axial movement of the shank tube (3), the control journal (20), which is fixedly connected to the handle (9), is mounted in a guiding manner in a helical guide track (21), wherein the helical guide track (21) is formed in a sleeve (22) which is securable in the adjusting wheel (18), and the adjusting wheel (18) is axially connected to the shank tube (3).

## Patentansprüche

1. Medizinisches Instrument mit einem Schaft (2), einem Werkzeug (7), das am distalem Ende (4) des Schaftes (2) angeordnet ist und aus zwei verschwenkbaren Maulteilen (5, 6) besteht, wobei die verschwenkbaren Maulteile (5, 6) zusätzlich in Bezug zu einer Längsachse (17) des Schaftes (2) abwinkelbar sind, und mit einer am proximalem Ende (8) des Schaftes (2) angeordneten Handhabe (9), wobei zwei separate Antriebe (10, 11) an der Handhabe (9) angeordnet sind, wobei ein erster Antrieb (10) zum Verschwenken eines ersten Maulteils (6) relativ zu einem zweiten Maulteil (5) als Griffteil (13) ausgebildet ist, der verschwenkbar an der Handhabe (9) gelagert ist und über eine erste axial verschiebbar im Schaft (2) gelagerte Zug-/Druckstange (15) mit dem ersten verschwenkbaren Maulteil (5) des Werkzeugs (7) in Wirkverbindung steht, wobei das Verschwenken der beiden Maulteile (5 und 6) relativ zueinander sowie das Abwinkeln der Maulteile (5 und 6) in Bezug zur Längsachse (17) des Schaftes (2) um dieselbe Schwenkachse (30) erfolgt, wobei die Schwenkachse (30) in Bezug zur Längsachse (17) des Schaftes (2) exzentrisch angeordnet ist,
**dadurch gekennzeichnet,**
**dass** der zweite Antrieb (11) zum Abwinkeln der zwei Maulteile (5, 6) in Bezug zur Längsachse (17) des Schaftes (2) über eine zweite axial verschiebbar im Schaft (2) gelagerte Stange (19) mit dem zweiten Maulteil (6) des Werkzeugs (7) in Wirkverbindung steht und wobei die Stange (19), die im Schaft (2) gelagert ist, an ihrem proximalen Ende fest mit der Handhabe (9) verbunden ist und einen festen Drehpunkt bildet, um den sich das zweite Maulteil (5) an ihrem distalen Ende verschwenken kann und, dass die Zug-/Druckstang (15) und die Stange (19) parallel nebeneinander in einem hohlen Schaftrohr (3) des Schaft (2) angeordnet sind und, dass beide parallel nebeneinander angeordneten Stangen (15 und 19) zusammen einen kreisförmigen Querschnitt aufweisen.

2. Medizinisches Instrument nach Anspruch 1, **dadurch gekennzeichnet, dass** das distale Ende (4) des Schaftes (2) gabelförmig ausgebildet aus zwei parallel und mit Abstand zueinander angeordneten Stegen (29) besteht, wobei die Schwenkachse (30) als Achsstift (31) ausgebildet ist, der in beiden Stegen (29) gelagert ist.

3. Medizinisches Instrument nach Anspruch 1, **dadurch gekennzeichnet, dass** die verschwenkbaren Maulteile (5 und 6) jeweils über Anlenkhebel (27) mit der jeweilig zugehörigen Zug-/Druckstange (15) und Stange (19) gekoppelt sind.

4. Medizinisches Instrument nach Anspruch 1, **dadurch gekennzeichnet, dass** jede, Zug-/Druckstange (15) und Stange (19), im Querschnitt halbkreisförmig ausgebildet ist.

5. Medizinisches Instrument nach Anspruch 1, **dadurch gekennzeichnet, dass** die Stange (19), die fest mit der Handhabe (9) verbunden ist, in einer Abflachung (24) der mit dem verschwenkbaren Griffteil (13) gekoppelten Zug-/Druckstange (15) gelagert ist.

6. Medizinisches Instrument nach einem der Ansprüche 1 bis 5, **dadurch gekennzeichnet, dass** der Antrieb (11) zum Abwinkeln der zwei Maulteile (5, 6) in Bezug zur Längsachse (17) des Schaftes (2) als Stellrad (18) ausgebildet ist, das um die Längsachse (17) des Schaftes (2) drehbar ist.

7. Medizinisches Instrument nach einem der Ansprüche 1 bis 6, **dadurch gekennzeichnet, dass** der Antrieb (11) zum Abwinkeln der zwei Maulteile (5, 6) in Bezug zur Längsachse (17) des Schaftes (2) über das relativ zur Handhabe (9) axialverschiebbar gelagerte Schaftrohr (3) mit den beiden Maulteilen (5, 6) in Wirkverbindung steht.

8. Medizinisches Instrument nach Anspruch 7, **dadurch gekennzeichnet, dass** das Schaftrohr (3) mit dem Stellrad (18) in Wirkverbindung steht, das wiederum über einen Steuerzapfen (20) mit der Handhabe (9) gekoppelt ist.

9. Medizinisches Instrument nach Anspruch 8, **dadurch gekennzeichnet, dass** zur Umwandlung der Rotationsbewegung des Stellrads (18) in die Axialbewegung des Schaftrohres (3) der fest mit der Handhabe (9) verbundene Steuerzapfen (20) in einer wendelförmige Führungsbahn (21) führend gelagert ist, wobei die Führungsbahn (21) in einer Hülse (22) ausgebildet ist, die im Stellrad (18) festlegbar ist und wobei das Stellrad (18) axial mit dem Schaftrohr (3) verbunden ist.

## Revendications

1. Instrument médical ayant une tige (2), un outil (7) qui est disposé sur l'extrémité distale (4) de la tige (2) et consiste en deux parties de mâchoire pouvant pivoter (5, 6), où les parties de mâchoire pouvant pivoter (5, 6) peuvent en outre être inclinées par rapport à un axe longitudinal (17) de la tige (2), et ayant une poignée (9) qui est disposée sur l'extrémité proximale (8) de la tige (2), où deux dispositifs d'entraînement (10, 11) séparés sont disposés sur la poignée (9), où un premier dispositif d'entraînement (10) pour faire pivoter une première partie de mâchoire (6) par rapport à la seconde partie de mâchoire (5) est conçu comme une partie de préhension (13) qui est montée à pivotement sur la poignée (9) et est reliée de manière active à la première partie de mâchoire pouvant pivoter (6) de l'outil (7) par le biais d'une première barre à va-et-vient (15) qui est montée d'une manière déplaçable axialement dans la tige (2), où le pivotement des deux parties de mâchoire (5 et 6) l'une par rapport à l'autre et l'inclinaison des parties de mâchoire (5 et 6) par rapport à l'axe longitudinal (17) de la tige (2) sont réalisés autour du même axe de pivot (30), où l'axe de pivot (30) est disposé de manière excentrée par rapport à l'axe longitudinal (17) de la tige (2)
**caractérisé en ce que** le second dispositif d'entraînement (11) pour incliner les deux parties de mâchoire (5, 6) par rapport à l'axe longitudinal (17) de la tige (2) est relié de manière active à la seconde partie de mâchoire (5) de l'outil (7) par le biais d'une seconde barre (19) qui est montée d'une manière axialement déplaçable dans la tige (2), et où,
la barre (19) qui est disposée dans la tige (2) est reliée de manière fixe à la poignée (9) sur son extrémité proximale et forme un pivot fixé autour duquel la seconde partie de mâchoire (5) peut pivoter sur son extrémité distale et **en ce que** la barre à va-et-vient (15) et la barre (19) sont disposées parallèlement côte à côte dans un tube de tige creux (3) de la tige (2), et **en ce que** les deux barres (15 et 19) disposées parallèlement côte à côte comprennent ensemble une section transversale circulaire.

2. Instrument médical selon la revendication 1, **caractérisé en ce que** l'extrémité distale (4) de la tige (2), conçue d'une manière bifurquée, consiste en deux bras (29) qui sont disposés parallèlement l'un à l'autre et à une distance l'un de l'autre, où l'axe de pivot (30) est réalisé sous forme d'un goujon d'axe (31) qui est monté dans les deux bras (29).

3. Instrument médical selon la revendication 1, **caractérisé en ce que** les parties de mâchoire (5 et 6) qui peuvent pivoter sont couplées dans chaque cas par le biais de leviers de couplage (27) avec la barre à va-et-vient (15) et la barre (19) associées respectivement.

4. Instrument médical selon la revendication 1, **caractérisé en ce que** chaque barre à va-et-vient (15) et barre (19) est conçue d'une manière semi-circulaire en section transversale.

5. Instrument médical selon la revendication 1, **caractérisé en ce que** la barre (19) qui est reliée de manière fixe à la poignée (2) est montée dans une zone plate (24) de la barre à va-et-vient (15) qui est couplée avec la partie de préhension (13) qui peut pivoter.

6. Instrument médical selon l'une des revendications 1 à 5, **caractérisé en ce que** le dispositif d'entraînement (11) pour incliner les deux parties de mâchoire (5, 6) par rapport à l'axe longitudinal (17) de la tige (2) est conçu comme une roue d'ajustement (18) qui peut tourner autour de l'axe longitudinal (17) de la tige (2).

7. Instrument médical selon l'une des revendications 1 à 6, **caractérisé en ce que** le dispositif d'entraînement (11) pour incliner les deux parties de mâchoire (5, 6) par rapport à l'axe longitudinal (17) de la tige (2) est relié de manière active aux deux parties de mâchoire (5, 6) de l'outil (7) par le biais du tube de tige (3) qui est monté d'une manière axialement déplaçable par rapport à la poignée (9).

8. Instrument médical selon la revendication 7, **caractérisé en ce que** le tube de tige (3) est relié de manière active à la roue d'ajustement (18), qui à son tour est couplée à la poignée (9) par le biais d'un tourillon de commande (20).

9. Instrument médical selon la revendication 8, **caractérisé en ce que** pour convertir le mouvement de rotation de la roue d'ajustement (18) en le mouvement axial du tube de tige (3), le tourillon de commande (20), qui est relié de manière fixe à la poignée (9), est monté de manière guidée dans une voie de guidage hélicoïdale (21), où la voie de guidage hélicoïdale (21) est formée dans une gaine (22) qui peut être fixée dans la roue d'ajustement (18), et la roue d'ajustement (18) est reliée axialement au tube de tige (3).
